# EUROPÄISCHE PATENTANMELDUNG

(11) **EP 0 916 355 A2**
(43) Veröffentlichungstag der Anmeldung: **19.05.1999**
(21) Anmeldenummer: 98120497.7
(22) Anmeldetag: 29.10.1998
(51) Int. Cl.: A61M 5/31

(54) **Injektionsset**

(30) Priorität: 07.11.1997 DE 29719826 U; 07.09.1998 DE 29816077 U
(71) Anmelder: Türk, Rudolf, Dr. med., 81735 München (DE)
(72) Erfinder: Türk, Rudolf, Dr. med., 81735 München (DE)
(74) Vertreter: Hofstetter, Alfons J., Dr.rer.nat.

(57) **Zusammenfassung**

Die Erfindung betrifft ein Injektionsset (10) mit mindestens einer sterilisierten Injektionsnadel (18) und mindestens jeweils einem darauf aufgeschobenen Nadelschutz (16), wobei das Injektionsset (10) zusätzlich mindestens ein Röhrchen (24) als Aufziehhilfe enthält und das oder die Röhrchen (24) auf jeweils einen Außenkonus (44) einer Spritzenspitze (26) einer medizinischen Spritze (28) aufsteckbar ist bzw. sind.

## Beschreibung

Die Erfindung betrifft ein Injektionsset, daß mindestens eine sterilisierte Injektionsnadel und mindestens einen darauf aufgeschobenen Nadelschutz enthält.

Übliche Injektionssets enthalten jeweils eine sterilisierte Injektionsnadel und einen darauf aufgeschobenen Nadelschutz. Injektionssets für medizinische Spritzen bestehen dabei üblicherweise aus einer äußeren Hülle, welche gewöhnlich zum einen Teil aus Tiefzieh-Kunststoff und zum anderen Teil aus einer aufreißbaren Papierhülle besteht. Darin verpackt ist die Injektionsnadel, bestehend aus der geschliffenen Metallnadel und dem Ansatzstück aus Kunststoff, welches einen Innenkonus aufweist, der auf den Außenkonus der Spritzenspitze paßt. Über die Nadel ist als Nadelschutz eine biegefeste Kunststoffhülle gestreift, die dem Schutz vor Beschädigungen der Nadelspitze und dem Schutz vor Verletzungen dient.

Verhält sich der Arzt "lege artis" d. h. nach dem Gesetz der ärztlichen Kunst, so nimmt er die Nadel samt Nadelschutz aus der Verpackung, nimmt eine Spritze aus der Verpackung, bricht die Ampulle mit der zu injizierenden Flüssigkeit, steckt die Spritze auf die Nadel, streift den Nadelschutz ab, zieht den Inhalt der Ampulle mit der Nadel auf, verwirft die gebrauchte Nadel, nimmt eine neue Nadel aus der Verpackung, steckt die Nadel samt Schutz auf die Spritze, streift den Schutz ab und injiziert den Spritzeninhalt.

Neben dieser ärztlicher Kunst entsprechenden Technik, sind mehrere fehlerhafte Varianten in Gebrauch, die sich alle dadurch auszeichnen, daß sie zum einen hohe Gesundheitsrisiken bergen, zum anderen unpraktisch sind, jedoch Zeit und Kosten sparen: Bei einer ersten Variante wird die zum Aufziehen des Ampulleninhalts verwendete Nadel auch zur Injektion verwendet. Diese Methode bringt zwei Nachteile mit sich. Zum einen ist, falls eine relativ dünne Injektionsnadel verwendet werden muß, das Aufziehen eines häufig nicht besonders dünnflüssigen Ampulleninhalts zeit- und kraftraubend. Zum anderen biegt sich häufig die sehr weiche Nadelspitze am Ampullenboden zu einem Widerhaken, was dem Arzt häufig verborgen bleibt. Als Folge ist die Injektion für den Patienten deutlich schmerzhafter, denn es werden sowohl beim Einstechen als auch beim Herausziehen wesentlich mehr Strukturen im Hautfettgewebe und Muskel, teilweise auch Nervenfasern oder Blutgefäße verletzt. Auch birgt die größere Verletzung ein höheres Risiko zu Blutungen und Spritzenabszessen und retrograd intravasaler Applikation.

Bei einer zweiten Variante wird zum Aufziehen des Ampulleninhalts nicht eine Nadel verwendet, sondern die Ampulle direkt mit der Spritzenspitze aufgezogen. Bei diesem Verfahren besteht ein hohes Gesundheitsrisiko für den Patienten. Der Spritzenkonus ist nämlich so kurz, daß er nur einen halben Zentimeter in die Ampulle reicht. Deshalb muß die Ampulle zusammen mit der Spritze gekippt werden, wodurch dann der Ampulleninhalt zum offenen Ende der Ampulle fließt. Oft tritt ein Tropfen des Ampulleninhalts trotz gleichzeitigen Aufziehens der Spritze aus dem offenen Ende der Ampulle aus, bleibt auf der unsterilen Außenseite der Ampulle hängen und wird beim weiteren Kippen der Ampulle gegen Ende des Aufziehvorgangs wieder in die Spritze mit eingesogen.

Hier liegt ein schwerer Hygienefehler des Arztes vor, welcher zu einem Großteil der Spritzenabszesse mit deren fatalen Folgen führt. Dennoch wird dieses Verfahren bei Ärzten und Helferinnen statistisch mehr angewandt als das Verfahren nach dem Gesetz der ärztlichen Kunst.

Demgemäß ist es Aufgabe der Erfindung, ein Injektionsset der eingangs genannten Art bereitzustellen, das kostengünstig ist und eine hygienisch einwandfreie Handhabung gewährleistet.

Diese Aufgabe wird erfindungsgemäß durch ein Injektionsset gemäß den Merkmalen des Anspruchs 1 gelöst.

Vorteilhafte Ausgestaltungen sind in den Unteransprüchen beschrieben.

Ein erfindungsgemäßes Injektionsset enthält zusätzlich mindestens ein Röhrchen als Aufziehhilfe, wobei das oder die Röhrchen auf jeweils einen Außenkonus einer Spritzenspitze einer medizinischen Spritze aufsteckbar ist. Wenn der Arzt das Injektionsset wie üblich ein kleines Stück aufbricht, steckt er zunächst den Konus der Spritzenspitze in das Röhrchen, welches elastisch ist und somit gut auf dem Spritzenkonus hält und dabei dicht schließt. Die Nadel selbst bleibt hygienisch einwandfrei, wenn mit der Spritze das Röhrchen aus dem Injektionsset herausgezogen wird. Der Aufziehvorgang selbst kann ohne das Kippen der Ampulle hygienisch einwandfrei und durch den im Vergleich zur Injektionsnadel großen Durchmesser des Röhrchens schnell und vollständig erfolgen. Die Erfindung macht sich zunutze, daß eine zum Aufziehen eines Ampulleninhalts verwendete Injektionsnadel Eigenschaften aufweisen würde, welche für diesen Vorgang nicht notwendig sind, beispielsweise eine geschliffene Edelstahlnadel, die darüberhinaus in einer Art und Weise mit einem Plastikkonus verschweißt ist, der auch hohem Injektionsdruck standhält. Durch Verwendung eines Röhrchens zum Aufziehen eines Ampulleninhalts sind die an die Aufziehvorrichtung gerichteten Anforderungen auf das wesentliche reduziert.

Üblicherweise umfaßt das erfindungsgemäße Injektionsset mindestens drei innerhalb einer gemeinsamen Verpackung angeordnete Elemente, nämlich die Injektionsnadel, den Nadelschutz und das Röhrchen als Aufziehhilfe. Es ist aber auch möglich, daß in einer weiteren vorteilhaften Ausgestaltung der Erfindung das sterilisierte Röhrchen separat oder zusammen mit der medizinischen Spritze steril aufbewahrt wird. Durch die separate Aufbewahrung des Röhrchens ist ein Höchstmaß an Hygiene gewährleistet. Wird das Röhrchen zusammen mit der medizinischen Spritze aufbewahrt und ist es jeweils auf der Spritzenspitze angeordnet, so entfällt vorteilhafterweise der Aufsteckvorgang des Röhrchens auf die medizinische Spritze. Dadurch wird eine weitere Kontaminationsmöglichkeit eliminiert und Zeit eingespart. Auch kann die Menge an Verpackungsmaterial minimiert werden, da das Röhrchen bereits der Spritzenverpackung beigepackt ist. Es ist auch möglich, daß die Spritze innerhalb der Verpackung des Injektionssets aufbewahrt wird.

In einer weiteren vorteilhaften Ausgestaltung der Erfindung ist das Röhrchen in einer Einzelverpackung angeordnet. Es kann daher innerhalb einer Spritzenverpackung angeordnet sein und mit einer abstreifbaren Kunststoff- oder Papierhülle versehen sein. Dadurch ist gewährleistet, daß beim Aufbrechen der Spritzenpackung Hygienefehler vermieden werden können, da das Röhrchen zusätzlich umhüllt ist.

In einer weiteren vorteilhaften Ausgestaltung der Erfindung ist das oder die Röhrchen in einem Spender angeordnet. Da mit dieser Ausgestaltungsart dem Arzt das Öffnen von sterilen Einzelverpackungen erspart bleibt und ihm jeweils auf Anforderung ein steriles Aufziehröhrchen zur Verfügung steht, ergibt sich hier eine besonders große Zeitersparnis.

In einer weiteren vorteilhaften Ausgestaltung der Erfindung ist das Röhrchen nach Art eines Kunststofftrinkhalms verformbar ausgebildet. Das Röhrchen weist dabei mindestens eine Biegefältelung auf. Eine derartige Biegefältelung erlaubt eine 180°-Biegung, so daß die Verpackung des Injektionssets, die Einzelverpackung oder die Spritzenpackung, in der das Röhrchen angeordnet ist, relativ klein gehalten werden können. Da der Kunststoff elastisch gewählt wird, ergibt sich ein guter Halt auf dem Spritzenkonus und ein dichter Verschluß. In dieser Ausgestaltung haftet das Röhrchen auch nicht so fest wie das Ansatzstück der Injektionsnadel auf dem Spritzenkonus, es ist leichter und mit geringerer Verletzungsgefahr zu entfernen. Darüberhinaus muß das Röhrchen auch nicht als Sondermüll entsorgt werden, weil vom Röhrchen selbst weder eine Infektionsgefahr durch vorherigen Patientenkontakt noch eine Verletzungsgefahr wie bei der geschärften Spitze der Nadel ausgeht.

Der Mehrkostenbedarf für das Röhrchen ist sehr gering, da das für Trinkhalme verwendete Material, welches handelsüblich ist, lebensmittelchemischen Ansprüchen genügt und somit hier eingesetzt werden kann. Zu berücksichtigen ist, daß die Sterilisation des Injektionssets erst nach Fertigstellung, d.h. nach Abpackung erfolgt.

Als besonders vorteilhaft hat sich erwiesen, wenn das Röhrchen im wesentlichen dieselbe Länge wie die Injektionsnadel samt aufgeschobenem Nadelschutz hat. Dadurch macht man sich die Steifheit des üblicherweise verwendeten Nadelschutzes zunutze, wodurch auch das vergleichsweise elastische Röhrchen vor Beschädigung geschützt wird und keine spezielle Verpackung, beispielsweise aus Hartkunststoff, für das Röhrchen notwendig ist.

Es kann jedoch auch vorgesehen werden, daß das Röhrchen von geringerer Länge ist als die Injektionsnadel samt aufgeschobenem Nadelschutz. Hierdurch wird sichergestellt, daß nach Entfernung des Röhrchen aus dem Injektionsset der Nadelschutz samt Injektionsnadel noch sicher in der aus Kunststoff und Papier gebildeten Verpackung des Injektionssets hält, somit nicht herausrutscht, was die Gefahr von Unsterilität mit sich bringen würde. Die Schutzwirkung des vergleichsweise steifen Nadelschutzes bleibt erhalten. Für besondere Anwendungen, beispielsweise in Kombination mit Injektionsnadeln, die aufgrund ihres Einsatzgebietes verhältnismäßig kurz ausgebildet sind, wie sie zum Beispiel für Injektionen dicht unter die Haut verwendet werden, kann auch vorgesehen werden, das Röhrchen länger als die Kombination aus Injektionsnadel und Nadelschutz auszubilden.

Es kann vorgesehen werden, den Durchmesser des Röhrchens über seine gesamte Länge hinweg konstant zu lassen, wobei er größer oder gleich groß ist als der Außendurchmesser des Endes des Spritzenkonus. Es kann jedoch auch vorgesehen werden, daß der Durchmesser des Röhrchens sich von dem einen für einen Kontakt mit einem Spritzenkonus vorgesehenen Ende zum gegenüberliegenden Ende hin verjüngt, wobei diese Verjüngung kontinuierlich oder stufenweise erfolgen kann. Für besondere Anwendungen kommt auch eine Vergrößerung des Durchmessers in Betracht.

In einer vorteilhaften Ausgestaltung besteht das Röhrchen aus durchsichtigem Material. Hierdurch läßt sich beim Aufziehen des Ampulleninhalts erkennen, ob das Röhrchen noch mit Injektionsflüssigkeit gefüllt ist oder nicht.

Im folgenden werden Ausführungsbeispiele unter Hinweis auf die beigefügten Zeichnungen näher beschrieben. Es stellen dar:
- Figur 1: zeigt in einer schematischen Darstellung ein erfindungsgemäßes Injektionsset gemäß einer ersten Ausführungsform;
- Figur 2: zeigt in einer schematischen Darstellung ein erfindungsgemäßes Injektionsset gemäß einer zweiten Ausführungsform;
- Figur 3a: zeigt eine Draufsicht auf einen Nadelschutz zur Verwendung in einem erfindungsgemäßen Injektionsset;
- Figur 3b: zeigt eine Draufsicht auf eine weitere Ausführungsform eines Nadelschutzes zur Verwendung in einem erfindungsgemaßen Injektionsset;
- Figur 4: zeigt eine schematische Darstellung einer Ausführungsform eines Röhrchens zur Verwendung in einem erfindungsgemäßen Injektionsset;
- Figur 5: zeigt eine schematische Darstellung eines Röhrchens zur Verwendung in einem erfindungsgemäßen Injektionsset in einer Einzelverpackung;
- Figur 6: zeigt in einer schematischen Darstellung ein Röhrchen zur Verwendung in einem erfindungsgemäßen Injektionsset in einer weiteren Einzelverpackung;
- Figur 7: zeigt in einer schematischen Darstellung ein Röhrchen zur Verwendung in einem erfindungsgemäßen Injektionsset in einer geöffneten Einzelverpackung;
- Figur 8: zeigt in einer schematischen Darstellung eine weitere Ausführungsform eines Röhrchens zur Verwendung in einem erfindungsgemäßen Injektionsset; und
- Figur 9: zeigt in einer schematischen Darstellung eine weitere Ausführungsform eines Röhrchens zur Verwendung in einem erfindungsgemäßen Injektionsset.

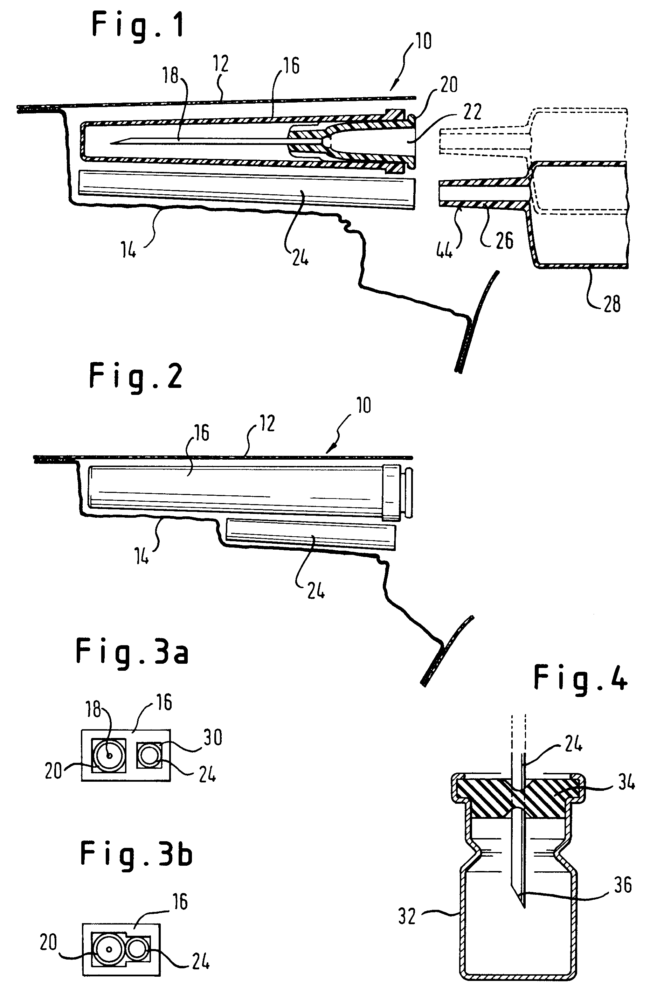

Figur 1 zeigt unter der Bezugsziffer 10 ein erfindungsgemäßes Injektionsset. Injektionssets umfassen gewöhnlich als Verpackung eine aus Papier bestehende Rückwand 12 sowie einen über den Inhalt der Packung geschrumpften Kunststoffilm 14. Es ist jedoch auch möglich, eine Vertiefung zur Aufnahme des Inhalts des Injektionssets 10 durch Tiefziehen eines Kunststoffs zu schaffen, wobei in einem weiteren Schritt die Rückwand 12 aufgebracht wird. Die Erfindung läßt sich mit allen in diesem Bereich bekannten Verpackungen realisieren.

Das Injektionsset umfaßt in herkömmlicher Weise eine in einem Nadelschutz 16, vorzugsweise aus biegesteifem Kunststoff, untergebrachte Injektionsnadel 18, die an dem der Nadelspitze gegenüberliegenden Ende ein Ansatzstück 20 mit einem Innenkonus 22 aufweist. Die Nadelspitze der Injektionsnadel 18 ist derart im Ansatzstück 20 befestigt, daß sie hohen Injektionsdrücken standhalten kann. Das Injektionsset 10 umfaßt weiterhin ein Röhrchen 24 als Aufziehhilfe. Dieses Röhrchen 24 ist ebenso wie die Injektionsnadel 18 samt Ansatzstück 20 steril und besteht vorzugsweise aus durchsichtigem Kunststoff. In der dargestellten Ausführungsform verjüngt sich der Durchmesser des Röhrchens 24 von der rechten Seite der Darstellung zur linken Seite. Es kann jedoch auch vorgesehen werden, daß das Röhrchen 24 über seine gesamte Länge einen konstanten Durchmesser aufweist. Der Durchmesser läßt sich auf den Durchmesser des Ampullenhalses abstimmen. Das Röhrchen 24 weist im wesentlichen dieselbe Länge auf wie die Injektionsnadel 18 samt aufgeschobenem Nadelschutz 16. Der Durchmesser des Röhrchens 24 muß groß genug sein, damit sich das Röhrchen über einen in Figur 1 schematisch als Querschnitt dargestellten Außenkonus 44 einer Spritzenspitze 26 schieben läßt bzw. die Spritzenspitze 26 in sich aufnehmen kann. Die Wandstärke des Röhrchens 24 ist im Hinblick auf geeignete Elastizität des Röhrchens 24 auf das verwendete Material abzustimmen.

Es kann jedoch auch vorgesehen werden, daß sich der Durchmesser des Röhrchens 24 von dem für den Kontakt mit der Spritzenspitze 26 vorgesehenen Ende zum gegenüberliegenden Ende hin verjüngt, wobei diese Verjüngung kontinuierlich oder stufenweise ausgestaltet sein kann. Für besondere Anwendungen, beispielsweise bei Spritzen mit sehr kleinem Durchmesser des Außenkonus 44 bzw. der Spritzenspitze 26, kommt auch eine Vergrößerung des Durchmessers in Betracht.

Das Röhrchen 24 liegt bei der dargestellten Ausführungsform unmittelbar auf dem Nadelschutz 16 auf, wobei die Steifheit des Nadelschutzes 16 auch das Röhrchen 24 vor Beschädigung schützt.

Es kann jedoch auch vorgesehen werden, daß das Röhrchen 24 in einem separaten Arbeitsgang einem herkömmlichen Injektionsset hinzugefügt wird, wobei sich dann zwischen Nadelschutz 16 und Röhrchen 24 eine Kunststoffolie (nicht dargestellt) befindet. Hierdurch wird zugleich sichergestellt, daß nach Öffnen der Verpackung des Sets 10 zur Benutzung des Röhrchens 24 der Rest der Verpackung, d.h. die Injektionsnadel 18 samt Nadelschutz 16, steril bleibt.

Zur Funktionsweise: Wie in Figur 1 schematisch dargestellt wird zunächst in einem ersten Arbeitsgang der Außenkonus 44 der Spritzenspitze 26 der Spritze 28 in das Röhrchen 24 eingeführt. Aufgrund der Elastizität des verwendeten Materials, vorzugsweise Kunststoff, bleibt das Röhrchen 24 zuverlässig und dicht abschließend auf der Spritzenspitze 26 stecken. Mit der mit dem Röhrchen 24 versehenen Spritzenspitze 26 wird nunmehr der Inhalt einer die Injektionsflüssigkeit enthaltenden Ampulle in den Hohlraum der Spritze 28 aufgesogen und anschließend das Röhrchen 24 von der Spritzenspitze 26 abgezogen. Da das Röhrchen 24 weder mit dem Patienten in Verbindung gekommen ist, noch über eine scharfkantige Spitze verfügt, kann dieses Röhrchen 24 problemlos entsorgt werden. Insbesondere muß dieses Röhrchen 24 nicht als Sondermüll entsorgt werden.

In einem zweiten Arbeitsgang wird nunmehr der mit der Ampullenflüssigkeit gefüllte Spritzenkörper 28 (siehe gestrichelte Darstellung in Figur 1 rechts oben) mit seiner Spritzenspitze 26 in das Ansatzstück 20 mit dem Innenkonus 22 der Injektionsnadel 18 in herkömmlicher Art und Weise eingeführt.

Nach Entfernen der auf der Spitzenspitze 26 haftenden Injektionsnadel 18 aus dem Nadelschutz 16 kann nunmehr vom Arzt dem Patienten die Injektionsflüssigkeit injiziert werden.

Figur 2 zeigt eine weitere Ausführungsform der Erfindung. In dieser Darstellung, wie auch den noch folgenden, bezeichnen gleiche Bezugszahlen gleiche Teile wie in Figur 1 und werden deshalb nicht nochmals beschrieben.

Bei dieser Ausführungsform ist das Röhrchen 24 von geringerer Länge als die Kombination aus Nadelschutz 16 und Injektionsnadel 18 in Figur 1. Hierdurch wird sichergestellt, daß auch nach Entfernen des Röhrchens 24 aus dem Injektionsset 10 die Injektionsnadel 18 samt Nadelschutz 16 sicher in der Verpackung hält, und somit bei Ablegen auf einer Ablage die sterilen Bestandteile des Injektionssets 10 nicht mit unsterilen Teilen der Umgebung in Berührung kommen. Die Länge des Röhrchens 24 läßt sich auf die Höhe der Ampulle, die die Injektionsflüssigkeit enthält, abstimmen.

Figuren 3a und 3b zeigen Weiterbildungen des Erfindungsgedankens. Während in Figur 3a der Nadelschutz 16 über eine weitere erste Kammer 30 zur Aufnahme des Röhrchens 24 verfügt, sind bei der in Figur 3b dargestellten Variante die beiden Kammern, d. h. die zur Aufnahme der Injektionsnadel 18 vorgesehene Kammer sowie die zur Aufnahme des Röhrchens 24 vorgesehene Kammer räumlich miteinander verbunden. Durch die Verwendung einer separaten Kammer für das Röhrchen 24 wird eine Beschädigung des Röhrchens 24 während des Transports des Injektionssets 10 sicher vermieden.

Gemäß einer nicht dargestellten Ausführungsform kann anstatt einer Kammer für das Rohrchen 24 auch vorgesehen werden, unter Verwendung eines Abstandshalters, der mit dem Nadelschutz 16 der Injektionsnadel 18 verbunden ist, einen sicheren Halt des Röhrchens 24 im Injektionsset 10 auch nach Öffnen des Sets 10 zu erzielen. Der Abstandshalter kann als mit dem Nadelschutz 16 verbundener Ring, vorzugsweise aus dem gleichen Material wie der Nadelschutz 16, ausgestaltet sein, durch den das Röhrchen 24 vor dem Aufziehen eines Ampulleninhalts gezogen werden kann.

Fig. 4 zeigt eine besondere Ausgestaltung des Röhrchens 24 wie sie zum Beispiel bei Ampullen 32 mit Gummistopfen 34 verwendet werden kann. Bei dieser Ausgestaltung ist das Röhrchen 24 mit einer Spitze 36 versehen, die in einfacher Weise durch schräges Abschneiden des Röhrchens 24 erhalten werden kann. Das Material des Röhrchens 24 resultiert in einer Steifheit, die geeignet ist, mit dem Röhrchen 24 den Gummistopfen 34, vorzugsweise an im Gummistopfen 34 ausgebildeten Stellen geringerer Stärke, zu durchdringen, um den Ampulleninhalt in die Spritze 28 aufzuziehen.

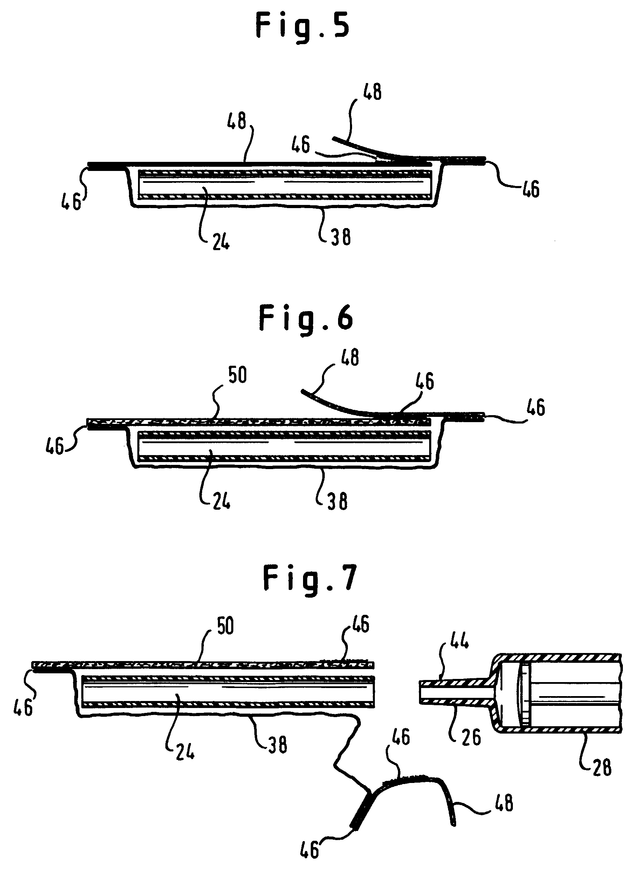

Figur 5 zeigt das Röhrchen 24, wobei es separat steril in einer Einzelverpackung 38 aufbewahrt wird. Die Einzelverpackung 38 besteht dabei aus einer Kunststoffverpackung, insbesondere einer einseitig tiefgezogenen weichen Kunststofffolie. Die Kunststoffverpackung 38 bildet einen Aufbewahrungsraum für das Röhrchen 24 aus, der an seiner nach oben offenen Seite mit einer Papierfolie 48 verschlossen ist. Die Papierfolie 48 setzt dabei seitlich an den Verklebungen 46 an und ist so mit der Kunststoffverpackung 38 lösbar befestigt. Dadurch entsteht eine peelbare Einzelverpackung. Da das Röhrchen 24 einzelverpackt ist, muß die Papierseite der Verpackung 38 eine peelbare Doppelung der Papierseite bzw. Papierfolie 48 aufweisen, da sonst das übliche Durchbrechen der Papierfolie 48 wegen der Flexibilität des Rohrchens 24 zu dessen Beschädigung führen würde. Es bietet sich auch an, eine Seite der Verpackung 38 zum Schutz des biegsamen Röhrchens 24 mit einer nicht knickbaren Verstärkung 50, wie dies in Figur 6 dargestellt ist, auszubilden.

In Figur 7 erkennt man, wie die Einzelverpackung 38 mittels der voneinander peelbaren Verklebungen 46 bzw. der Papierfolie 48 geöffnet werden kann, so daß das Röhrchen 24 teilweise freiliegt, um mit einem Ende auf den Außenkonus 44 der Spritzenspitze 26 der Spritze 28 aufgeschoben werden zu können. Man erkennt auch, daß der überwiegende Teil des Röhrchens 24 noch immer in der Kunststoffverpackung 38 verweilt und somit vor Kontaminationen geschützt ist. Die beschriebenen Einzelverpackungen 38 lassen sich auch zur gemeinsamen Aufbewahrung des Injektionssets 10 bestehend aus der Injektionsnadel 18, dem Nadelschutz 16 und dem Röhrchen 24 verwenden.

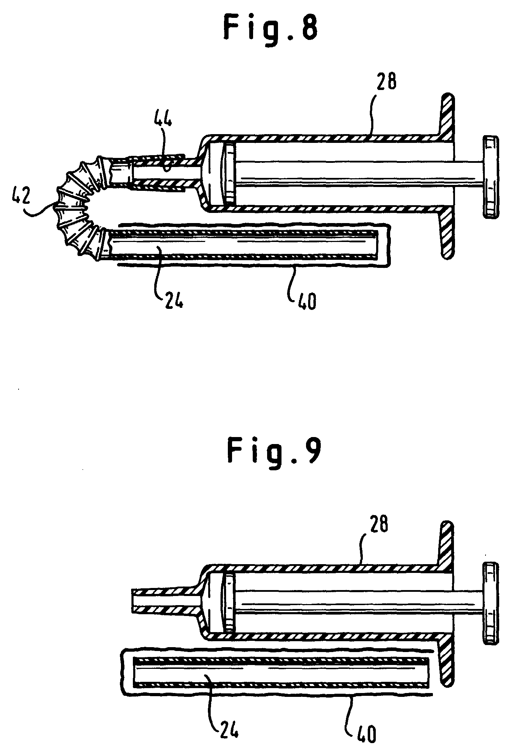

Figur 8 zeigt eine weitere Ausführungsgform des Röhrchens 24. Man erkennt, daß das Aufziehröhrchen 24 nach Art eines Kunststofftrinkhalmes verformbar ist und eine Biegefältelung 42 aufweist. Die Biegefältelung 42 ist dabei über mindestens 180° biegbar. Man erkennt, daß in dem gezeigten Ausführungsbeispiel das Röhrchen 24 bereits auf dem Außenkonus 44 der Spritzenspitze 26 der Spritze 28 angeordnet ist. Beide Elemente liegen in einer gemeinsamen Spritzenverpackung (nicht dargestellt). Man erkennt, daß das Röhrchen 24 in seinem geraden Teil mit einer abstreifbaren sterilen Schutzhülle 40 versehen ist, welche mit der Spritze 28 zusammen aus der nicht dargestellten Gesamtverpackung genommen wird und dazu dient, die Biegefältelung 42 für den Aufziehvorgang gerade zu strecken ohne das Röhrchen 24 zu kontaminieren. Direkt vor dem Aufziehen der Spritze 28 wird dann die Schutzhülle 40 abgestreift.

Figur 9 zeigt ebenfalls ein Röhrchen 24, daß innerhalb einer Spritzenverpackung (nicht dargestellt) angeordnet ist. Das Röhrchen 24 ist dabei mit der Schutzhülle 40 versehen. Im Anwendungsfalle bricht der Arzt die nicht dargestellte gemeinsame Verpackung und entnimmt die Spritze 28, wobei er anschließend den Außenkonus 44 in das noch in der Verpackung liegende Röhrchen 24 einführt. Anschließend wird die Schutzhülle 40 abgestreift, so daß über das Röhrchen 24 der Inhalt einer Ampulle in die Spritze 28 übergeführt werden kann.

## Patentansprüche

1. Injektionsset (10) mit mindestens einer sterilisierten Injektionsnadel (18) und mindestens jeweils einem darauf aufgeschobenen Nadelschutz (16),
**dadurch gekennzeichnet,**
daß das Injektionsset (10) zusätzlich mindestens ein Röhrchen (24) als Aufziehhilfe enthält, wobei das oder die Röhrchen (24) auf jeweils einen Außenkonus (44) einer Spritzenspitze (26) einer medizinischen Spritze (28) aufsteckbar ist.

2. Injektionsset nach Anspruch 1,
**dadurch gekennzeichnet,**
daß das Röhrchen (24) separat oder zusammen mit der medizinischen Spritze (28) steril aufbewahrt wird.

3. Injektionsset nach Anspruch 2,
**dadurch gekennzeichnet,**
daß das Röhrchen (24) in einer Einzelverpackung (38) angeordnet ist.

4. Injektionsset nach Anspruch 2,
**dadurch gekennzeichnet,**
daß das Röhrchen (24) innerhalb einer Spritzenverpackung (50) angeordnet ist.

5. Injektionsset nach einem der vorhergehenden Ansprüche,
**dadurch gekennzeichnet,**
daß das Röhrchen (24) mit einer abstreifbaren Schutzhülle (40) aus Kunststoff oder Papier versehen ist.

6. Injektionsset nach einem der vorhergehenden Ansprüche,
**dadurch gekennzeichnet,**
daß das Röhrchen (24) in einem Spender angeordnet ist.

7. Injektionsset nach einem der vorhergehenden Ansprüche,
**dadurch gekennzeichnet,**
daß das Röhrchen (24) nach Art eines Kunststofftrinkhalms verformbar ist und mindestens eine Biegefältelung (42) aufweist.

8. Injektionsset nach einem der vorhergehenden Ansprüche,
**dadurch gekennzeichnet,**
daß das Röhrchen (24) im wesentlichen dieselbe Länge wie die Injektionsnadel (18) samt aufgeschobenem Nadelschutz (16) hat.

9. Injektionsset nach einem der Ansprüche 1 bis 7,
**dadurch gekennzeichnet,**
daß das Röhrchen (24) kürzer oder länger als die Injektionsnadel (18) samt aufgeschobenem Nadelschutz (16) ist.

10. Injektionsset nach einem der vorhergehenden Ansprüche,
**dadurch gekennzeichnet,**
daß der Durchmesser des Röhrchens (24) über seine Länge hinweg konstant ist, wobei er größer oder gleich groß ist als der Außendurchmesser des Endes des Außenkonus (44) der Spritzenspitze (26).

11. Injektionsset nach einem der Ansprüche 1 bis 9,
**dadurch gekennzeichnet,**
daß der Durchmesser des Röhrchens (24) sich von dem für einen Kontakt mit dem Außenkonus (44) vorgesehenen Ende zum gegenüberliegenden Ende hin verjüngt.

12. Injektionsset nach Anspruch 11,
**dadurch gekennzeichnet,**
daß sich das Röhrchen (26) kontinuierlich oder stufenweise verjüngt.

13. Injektionsset nach einem der vorhergehenden Ansprüche,
**dadurch gekennzeichnet,**
daß das Röhrchen (24) durchsichtigt ist.

14. Injektionsset nach einem der vorhergehenen Ansprüche,
**dadurch gekennzeichnet,**
daß das Röhrchen (24) unmittelbar auf dem Nadelschutz (16) aufliegt.

15. Injektionsset nach einem der Ansprüche 1 bis 13,
**dadurch gekennzeichnet,**
daß sich zwischen dem Röhrchen (24) und dem Nadelschutz (16) eine Kunststoffolie befindet.

16. Injektionsset nach einem der vorhergehenden Ansprüche,
**dadurch gekennzeichnet,**
daß ein Ende des Röhrchens (24) über eine Spitze verfügt.

17. Injektionsset nach einem der vorhergehenden Ansprüche,
**dadurch gekennzeichnet,**
daß das Röhrchen (24) durch eine Haltevorrichtung im Injektionsset (10) fixiert ist.

18. Injektionsset nach Anspruch 17,
**dadurch gekennzeichnet,**
daß die Haltevorrichtung in einer im Nadelschutz (16) ausgebildeten zweiten Kammer (54) besteht.

19. Injektionsset nach Anspruch 17
**dadurch gekennzeichnet,**
daß die Haltevorrichtung einen Haltering umfaßt, der vorzugsweise mit dem Nadelschutz (16) verbunden ist.
